# EUROPEAN PATENT SPECIFICATION

(11) **EP 3 492 088 B1**
(45) Date of publication and mention of the grant of the patent: **01.09.2021**
(21) Application number: 17834183.0
(22) Date of filing: 21.07.2017
(51) Int. Cl.: A61K 33/00, A61K 9/72, A61K 47/02, A61P 9/00

(54) **MEDICINAL COMPOSITION FOR IMPROVING AND/OR STABILIZING CIRCULATORY DYNAMICS AFTER ONSET OF HEMORRHAGIC SHOCK**
MEDIZINISCHE ZUSAMMENSETZUNG ZUR VERBESSERUNG UND/ODER STABILISIERUNG DER KREISLAUFDYNAMIK NACH DEM EINTRITT EINES HÄMORRHAGISCHEN SCHOCKS
COMPOSÉ MÉDICINAL POUR AMÉLIORER ET/OU STABILISER LA CIRCULATION APRÈS UN CHOC HÉMORRAGIQUE.

(30) Priority: 29.07.2016 JP 2016150761
(43) Date of publication of application: 05.06.2019
(73) Proprietor: Keio University, Tokyo 108-8345 (JP); Taiyo Nippon Sanso Corporation, Tokyo 142-8558 (JP)
(72) Inventor: SUZUKI, Masaru, Tokyo 160-8582 (JP); MATSUOKA, Tadashi, Tokyo 160-8582 (JP); TAMURA, Tomoyoshi, Tokyo 1608582 (JP); HAYASHIDA, Kei, Tokyo 160-8582 (JP); SANO, Motoaki, Tokyo 160-8582 (JP)
(74) Representative: Bridle, Andrew Barry
(86) International application number: PCT/JP2017/026431
(87) International publication number: WO 2018/021175

(56) References cited:
- WO-A1-2014/024984
- KOHAMA KEISUKE et al.: "Hydrogen inhalation protects against acute lung injury induced by hemorrhagic shock and resuscitation", Surgery, vol. 158, no. 2, August 2015 (2015-08), pages 399-407, XP055569864,
- SHEN MEIHUA et al.: "A review of experimental studies of hydrogen as a new therapeutic agent in emergency and critical care medicine", MEDICAL GAS RESEARCH, vol. 4, no. 17, 8 November 2014 (2014-11-08), pages 1-8, XP021203691, ISSN: 2045-9912
- YOTARO SHINOZAWA et al.: "6. PATHOPHYSIOLOGIES OF ISCHEMIC/REPERFUSION INJURIES ASSOCIATING WITH HEMORRHAGIC SHOCK AND UP-TO-DATA TREATMENT", Journal of Japan Surgical Society, vol. 104, no. 12, 1 December 2003 (2003-12-01), pages 835-839, XP9513347,
- TADASHI MATSUOKA et al.: "Rat Shukketsusei Shock Model ni Okeru Suiso Gas Kyunyu no Seizonritsu Kaizen Koka ni Tsuite", Journal of Abdominal Emergency Medicine, vol. 37, no. 2, 2 February 2017 (2017-02-02), page 222, XP9513319,
- MATSUOKA TADASHI et al.: "Hydrogen gas inhalation inhibits progression to the ''irreversible", Journal of Trauma and Acute Care Surgery, 10 August 2017 (2017-08-10), XP9513321, [retrieved on 2017-06-20]

## Description

### Technical Field

The present invention relates to a pharmaceutical composition for improving and/or stabilizing circulatory dynamics after onset of hemorrhagic shock.

### Background Art

Hemorrhagic shock is a symptom that results from losing a large amount of blood in the body due to some kind of factor such as injury or extreme dehydration, evokes symptoms such as increase in pulse rate, decline in body temperature and blood pressure, respiratory failure, and clouding of consciousness, and when left without giving some kind of treatment, ultimately leads to death. Hemorrhagic shock is said to be the cause of about half of deaths during various surgeries.

It is well-known that hemostasis and blood transfusion therapy are the basic therapies for hemorrhagic shock. However, in reality, there are numerous situations where blood transfusion cannot be promptly given to a patient who has developed hemorrhagic shock. Needless to say, blood transfusion requires suitably stored and maintained blood, and for example when one has developed hemorrhagic shock caused by an injury at a location distant from a medical care facility or a location with difficult access by an ambulance, blood transfusion cannot be given promptly. Many of the instances where the cause of injury is by an unforeseen accident will correspond to such a case.

Accordingly, for hemorrhagic shock, it is extremely important to stabilize patient condition and maintain survival until basic therapy is given. Currently, fluid therapy which supplements lost blood with saline is employed for maintaining the patient after onset of hemorrhagic shock. However, situations where even fluid therapy is difficult to carry out are conceivable, and moreover, symptomatic therapy by fluid therapy does not necessarily achieve a satisfactory result in terms of extending the survival time of a hemorrhagic shock patient.

It has been reported thus far that hydrogen is effective for particular symptoms attributed to hemorrhagic shock. For example, it has been reported that hydrogen gas inhalation has a protective effect on acute lung disorder induced by hemorrhagic shock and the subsequent resuscitation by blood transfusion (Non-Patent Literature 1).

It has also been reported that intestinal disorder and lung disorder induced by hemorrhagic shock is improved by administration of a transfusion containing hydrogen (Non-Patent Literatures 2 and 3).

### Citation List

[Non-Patent Literature 1] Kohama K, et al. Hydrogen inhalation protects against acute lung injury induced by hemorrhaig shock and resuscitation. Surgery 2015; 158: 399.
[Non-Patent Literature 2] Zunmin Du, Three hydrogen-rich solutions protect against intestinal injury in uncontrolled hemorrhagic shock. Int J Clin Exp Med 2015; 8 (5): 7620-7626
[Non-Patent Literature 3] Zunmin Du, MB, et al., Effects of three hydrogen-rich liquids on hemorrhagic shock in rats. JOURNAL OF SURGICAL RESERACH 193 (2015) 377-382

### Summary of the Invention

### Problems to be Solved by the Invention

The object of the present invention is to provide a pharmaceutical composition that can improve the survival time and the survival rate of a patient after onset of hemorrhagic shock.

### Means for Solving the Problems

Although it is known that exacerbation of various conditions is caused by hemorrhagic shock, it is not necessarily clear at this point the exacerbation of which condition is mainly attributed to death after onset of hemorrhagic shock.

The present inventors, in the process of verifying the survival time or the survival rate after onset of hemorrhagic shock, surprisingly found that the exacerbation of circulatory dynamics is deeply involved in the survival time and the survival rate after onset of hemorrhagic shock.

The present inventors further surprisingly found that hydrogen gas improves and/or stabilizes circulatory dynamics after onset of hemorrhagic shock, and can thereby improve the survival time and the survival rate.

The present invention is based on the above findings, and encompasses the following characteristics.
[1] A gaseous pharmaceutical composition for improving and/or stabilizing circulatory dynamics after onset of hemorrhagic shock, characterized in that said pharmaceutical composition comprises hydrogen gas.
[2] The pharmaceutical composition according to [1], characterized in that said pharmaceutical composition is administered when blood transfusion therapy cannot be given to a subject.
[3] The pharmaceutical composition according to [1] or [2], characterized in that said subject is a subject to which fluid therapy is given.
[4] The pharmaceutical composition according to [3], characterized in that said fluid therapy is given prior to, at the same time with, or after administering said composition.
[5] The pharmaceutical composition according to any one of [1] to [4], characterized in that the survival rate after hemorrhagic shock is improved by administration of said pharmaceutical composition.
[6] The pharmaceutical composition according to any one of [1] to [4], characterized in that the survival time after hemorrhagic shock is extended by administration of said pharmaceutical composition.
[7] The pharmaceutical composition according to any one of [1] to [6], characterized in that said improvement and/or stabilization of circulatory dynamics is the maintenance of blood pressure or the maintenance of blood supply to an essential organ based on improvement and/or stabilization of cardiac function or vascular function.
[8] The pharmaceutical composition according to any one of [1] to [7], characterized in that said improvement and/or stabilization of cardiac function or vascular function is suppression of cell death in blood vessels, the heart, or other organs under a hypoxic condition.
[9] The pharmaceutical composition according to any one of [1] to [8], characterized in that said pharmaceutical composition further comprises oxygen gas.
[10] The pharmaceutical composition according to any one of [1] to [9], characterized in that said pharmaceutical composition further comprises an inert gas.
[11] The pharmaceutical composition according to any one of [1] to [10], characterized in that the concentration of said hydrogen in said pharmaceutical composition is 0.1% - 4.0% (v/v).
[12] The pharmaceutical composition according to any one of [1] to [11], characterized in that the concentration of said hydrogen in said pharmaceutical composition is 1.0% - 2.0% (v/v).
[13] The pharmaceutical composition according to any one of [1] to [12], characterized in that said hydrogen gas is provided by a hydrogen gas generation apparatus or a container that contains hydrogen gas.
[14] The pharmaceutical composition according to [13], characterized in that said hydrogen gas generation apparatus comprises a hydrogen generation means by water electrolysis.
[15] The pharmaceutical composition according to [13], characterized in that said container contains a mixed gas of hydrogen and nitrogen gases.
[16] The pharmaceutical composition according to any one of [13] to [15], characterized in that said hydrogen gas generation apparatus or said container further comprises a control means for monitoring and adjusting the amount of hydrogen gas supplied to the subject, on said device or the container itself or a piping connected to said device or container.
[17] A gaseous pharmaceutical composition for promoting improvement or stabilization of circulatory dynamics after hemorrhagic shock by fluid therapy, characterized in that:
   said pharmaceutical composition comprises hydrogen gas, and
   the survival rate after hemorrhagic shock is improved by administration of said pharmaceutical composition and/or the survival time after hemorrhagic shock is extended by administration of said pharmaceutical composition.
[18] A gaseous pharmaceutical composition for extending the survival time after hemorrhagic shock, characterized in that said pharmaceutical composition comprises hydrogen gas.
[19] A gaseous pharmaceutical composition for improving the survival rate after hemorrhagic shock, characterized in that said pharmaceutical composition comprises hydrogen gas.

An invention of any combination of one or more of the aspects of the present invention listed above is also encompassed by the scope of the present invention.

### Effects of the Invention

According to the present invention, a pharmaceutical composition that improves and/or stabilizes circulatory dynamics after onset of hemorrhagic shock is provided. With the effect of improving and/or stabilizing circulatory dynamics after onset of hemorrhagic shock, the pharmaceutical composition of the present invention can increase the survival rate or extend the survival time of a subject after onset of hemorrhagic shock.

### Brief Description of the Drawings

Figure 1 shows the overview of the tests performed in the Examples of the present invention.
Figure 2 shows the comparison of survival rate between the hydrogen inhalation group and the control group after initiation of resuscitation following hemorrhagic shock.
Figure 3 shows the change over time of systolic blood pressure in the hydrogen inhalation group and the control group after initiation of resuscitation following hemorrhagic shock.
Figure 4 shows the change over time of mean blood pressure in the hydrogen inhalation group and the control group after initiation of resuscitation following hemorrhagic shock.
Figure 5 shows the change over time of systolic blood pressure in the hydrogen inhalation group and the control group after initiation of resuscitation following hemorrhagic shock.
Figure 6 shows the change over time of heart rate (pulse) in the hydrogen inhalation group and the control group after initiation of resuscitation following hemorrhagic shock.
Figure 7 shows the comparison of lactic acid value in the hydrogen inhalation group and the control (Ctrl) group at 120 minutes after initiation of resuscitation following hemorrhagic shock (initiation point of observation phase). Note that the black circle in the figure indicates a deceased individual.
Figure 8 shows the comparison of pH value in the hydrogen inhalation group and the control (Ctrl) group at 120 minutes after initiation of resuscitation following hemorrhagic shock (initiation point of observation phase). Note that the black circle in the figure indicates a deceased individual.
Figure 9 shows the comparison of HCO3 value in the hydrogen inhalation group and the control (Ctrl) group at 120 minutes after initiation of resuscitation following hemorrhagic shock (initiation point of observation phase). Note that the black circle in the figure indicates a deceased individual.
Figure 10 shows the comparison of BE (base excess) value in the hydrogen inhalation group and the control (Ctrl) group at 120 minutes after initiation of resuscitation following hemorrhagic shock (initiation point of observation phase). Note that the black circle in the figure indicates a deceased individual.
Figure 11 shows the comparison of Na value in the hydrogen inhalation group and the control (Ctrl) group at 120 minutes after initiation of resuscitation following hemorrhagic shock (initiation point of observation phase). Note that the black circle in the figure indicates a deceased individual.
Figure 12 shows the comparison of K value in the hydrogen inhalation group and the control (Ctrl) group at 120 minutes after initiation of resuscitation following hemorrhagic shock (initiation point of observation phase). Note that the black circle in the figure indicates a deceased individual.
Figure 13 shows the comparison of hemoglobin (Hb) value in the hydrogen inhalation group and the control (Ctrl) group at 120 minutes after initiation of resuscitation following hemorrhagic shock (initiation point of observation phase). Note that the black circle in the figure indicates a deceased individual.
Figure 14 shows the comparison of PaO2 value in the hydrogen inhalation group and the control (Ctrl) group at 120 minutes after initiation of resuscitation following hemorrhagic shock (initiation point of observation phase). Note that the black circle in the figure indicates a deceased individual.
Figure 15 shows the comparison of PaCO2 value in the hydrogen inhalation group and the control (Ctrl) group at 120 minutes after initiation of resuscitation following hemorrhagic shock (initiation point of observation phase). Note that the black circle in the figure indicates a deceased individual.
Figure 16 shows the change over time of left ventricular end-diastolic pressure (LVEDP) in the hydrogen inhalation group and the control group after initiation of resuscitation following hemorrhagic shock.
Figure 17 shows the change over time of positive dP/dt in the hydrogen inhalation group and the control group after initiation of resuscitation following hemorrhagic shock.
Figure 18 shows the change over time of negative dP/dt in the hydrogen inhalation group and the control group after initiation of resuscitation following hemorrhagic shock.
Figure 19 shows the result of the survival rate by hydrogen inhalation after onset of hemorrhagic shock.

### Description of Embodiments

The embodiments of the present invention will now be specifically described below.

The present invention is in part based on the findings that the exacerbation of circulatory dynamics after onset of hemorrhagic shock is the main cause of death attributed to hemorrhagic shock, as well as that said circulatory dynamics after onset of hemorrhagic shock can be improved or stabilized by hydrogen gas.

"Improvement of circulatory dynamics" as used herein refers to changing to the direction towards a normal state the circulatory dynamics deviated from a normal state attributed to hemorrhagic shock.

In contrast, "stabilization of circulatory dynamics" can mean maintaining the circulatory dynamics at a state close to a normal state or suppressing further exacerbation.

Accordingly, in one aspect, the present invention relates to a pharmaceutical composition for improving and/or stabilizing circulatory dynamics after onset of hemorrhagic shock. By improving and/or stabilizing the "circulatory dynamics" described below, the pharmaceutical composition of the present invention can improve the survival rate of a subject after onset of hemorrhagic shock, or can extend the survival time of a subject after onset of hemorrhagic shock.

"After onset of hemorrhagic shock" as used herein is intended to exclude prior to the initiation of hemorrhagic shock, and can include any and all time points where exacerbation of circulatory dynamics attributed to hemorrhagic shock is recognized. Accordingly, a subject "after onset of hemorrhagic shock" includes a subject having a hemorrhagic shock, as well as a subject after withdrawal from hemorrhagic shock.

The "survival rate after onset of hemorrhagic shock" as used herein may refer to the survival rate attributed to hemorrhagic shock, as well as the survival rate at any timepoint after onset of hemorrhagic shock. Accordingly, the "survival rate after onset of hemorrhagic shock is improved" may mean that the survival rate attributed to hemorrhagic shock is increased compared to when the pharmaceutical composition of the present invention is not administered, as well as that the survival rate at any timepoint after onset of hemorrhagic shock is increased compared to when the pharmaceutical composition of the present invention is not administered. The timepoint after onset of hemorrhagic shock is not particularly limited, and for example can be the initiation point of the curative treatment (such as 30 minutes, 1 hour, 2 hours, 4 hours, 8 hours, 12 hours, 16 hours, or 24 hours after onset of hemorrhagic shock), or any timepoint after the initiation of the curative treatment.

"Circulatory dynamics" refers to the state of circulatory function, and is known to be generally determined by three factors: cardiac function, blood vessels, and circulating blood volume. In one embodiment of the present invention, "improvement and/or stabilization of circulatory dynamics" comprises the maintenance of blood pressure or the maintenance of blood supply to an essential organ based on improvement and/or stabilization of cardiac function or vascular function.

Improvement of cardiac function includes a direct effect by improvement of diastolic function (myocardial extensibility) and an indirect effect by improvement of vascular function (increase in preload: increase in vein perfusion volume). In the present invention, improvement and/or stabilization of cardiac function or vascular function particularly includes an increase in vein perfusion volume (increase in preload), an improvement of myocardial extensibility (improvement of cardiac diastole), and an increase in cardiac output volume.

In one embodiment of the present invention, "improvement and/or stabilization of cardiac function or vascular function" includes suppression of cell death in blood vessels, the heart, or other organs under a hypoxic condition. The production of a substance from cells that exacerbates circulatory dynamics is thereby suppressed. Said suppression effect can be confirmed by improvement of any biological parameter, and such a parameter includes, but is not limited to, blood lactic acid value, pH, HCO3 concentration, BE (base excess), potassium concentration, and the like. In a preferred embodiment of the present invention, at least two, more preferably at least three, and most preferably all four of said circulatory failure parameters are improved in the direction towards a normal value.

In one embodiment of the present invention, "improvement and/or stabilization of circulatory dynamics" includes increase in resistance against hemorrhage. "Increase in resistance against hemorrhage" as used herein may mean that the state of a subject with the same amount of hemorrhage (such as any circulatory dynamics parameter) is closer to normal, the survival rate of a subject with the same amount of hemorrhage is increased, or the survival time of a subject with the same amount of hemorrhage is extended, compared to when the pharmaceutical composition of the present invention is not administered. Alternatively, "increase in resistance against hemorrhage" as used herein refers to the increase in the amount of hemorrhage necessary to reach a particular state (such as any circulatory dynamics parameter) compared to when the pharmaceutical composition of the present invention is not administered.

In a preferred embodiment of the present invention, "improvement and/or stabilization of circulatory dynamics" includes improvement and/or stabilization of blood pressure and improvement and/or stabilization of cardiovascular function.

In one embodiment, the pharmaceutical composition of the present invention is administered when blood transfusion therapy cannot be given. In other words, in one embodiment, the pharmaceutical composition of the present invention can be used with the objective to extend the survival time of a subject to whom basic therapy cannot be given. This is achieved by virtue of the pharmaceutical composition of the present invention with the effect of improving and/or stabilizing circulatory dynamics. Note that it is not excluded to use the pharmaceutical composition of the present invention in combination with blood transfusion therapy. Although the pharmaceutical composition of the present invention may be more useful on a subject to whom blood transfusion therapy cannot be given as described above, a more rapid recovery from hemorrhagic shock can be expected by using a basic therapy such as blood transfusion therapy in combination.

In one embodiment, the pharmaceutical composition of the present invention is used in combination with other therapies for improving or stabilizing circulatory dynamics after onset of hemorrhagic shock. Such other therapies can include, but are not limited to, fluid therapy. Accordingly, in another aspect, the pharmaceutical composition of the present invention can also be used as a pharmaceutical composition for promoting improvement or stabilization of circulatory dynamics after onset of hemorrhagic shock by fluid therapy.

When the pharmaceutical composition of the present invention and fluid therapy are used in combination, the timing for giving the fluid therapy is not particularly limited, and may be given prior to, the same time with, or after administration of the pharmaceutical composition of the present invention.

The pharmaceutical composition of the present invention is characterized in that it is a gaseous pharmaceutical composition comprising hydrogen gas. Moreover, since the pharmaceutical composition of the present invention is a gaseous pharmaceutical composition, it is characterized to be continuously administered to a human patient over a certain period of time. In the present invention, the hydrogen atom may be any of all of its isotopes, i.e. protium (P or ¹H), deuterium (D or ²H), and tritium (T or ³H). Accordingly, P₂, PD, PT, DT, D₂, and T₂ may be included as molecular hydrogen. In a preferred aspect of the present invention, 99% or more of the hydrogen gas included in the pharmaceutical composition of the present invention is the natural molecular hydrogen P₂.

The pharmaceutical composition of the present invention can further comprise oxygen gas. Oxygen gas may be premixed with hydrogen gas and exist in a mixed gas form, or may be mixed with hydrogen gas immediately before administration to a subject or at the time of administration.

The pharmaceutical composition of the present invention can further comprise an inert gas. Inert gas is used with the objective to prevent explosion and to adjust the concentration of hydrogen or oxygen gases, and accordingly may exist in a mixed gas form with hydrogen and/or oxygen gases. The inert gas that can be used in the pharmaceutical composition of the present invention can be, but is not limited to, nitrogen gas, helium gas, argon gas, and the like. In one embodiment of the present invention, inexpensive nitrogen gas is used as the inert gas.

In the pharmaceutical composition of the present invention, the concentration range of hydrogen gas can be, but is not limited to, for example any concentration between 0.1 - 4.0% (v/v) . The lower limit of the hydrogen gas concentration is that set as the lower limit of the concentration which can exert an effect of improving and/or stabilizing circulatory dynamics after onset of hemorrhagic shock. Accordingly, the minimum concentration that can improve and/or stabilize circulatory dynamics after onset of hemorrhagic shock can be appropriately set according to the severity of the patient, the injury etc. which was the cause of hemorrhagic shock, the time from onset of hemorrhage shock to initiation of administration, sex, age, and the like. In one embodiment, the lower limit of hydrogen gas can be selected to be between 0.1 - 1.0%, for example 0.5%. On the other hand, the upper limit of hydrogen gas concentration is set with respect to safety, since the lower explosion limit of hydrogen in air is 4%. Accordingly, the upper limit of hydrogen gas can be selected to be any concentration that is 4% or less as long as safety is guaranteed, for example 3.0%, 2.5%, or 2.0% and the like.

In the pharmaceutical composition of the present invention, assuming that the hydrogen gas concentration is 0.1 - 4.0% (v/v), the concentration of oxygen gas can be in the range of 21% - 99.9% (v/v).

In the pharmaceutical composition of the present invention, the concentration of the inert gas is set at a range that suitably maintains the concentration of hydrogen and/or oxygen gases, as well as yields the explosion-proof effect of these gases. Accordingly, for the concentration of the inert gas, those skilled in the art can appropriately set a suitable concentration according to the concentration of hydrogen and/or oxygen gases used. The concentration of such inert gas may be, for example when the inert gas is nitrogen gas, for example any concentration in the range of 0 - 78.9% (v/v).

Note that the concentration of gases used throughout the present specification is shown as percentage of content at 20°C, 101.3 kPa.

In the pharmaceutical composition of the present invention, other gases in the atmosphere such as carbon dioxide, air, or anesthetic gas and the like may be further comprised as long as the effects of the invention by hydrogen gas is not impaired.

The pharmaceutical composition of the present invention can be administered to a subject for example by inhalation with an inhalation means. An example of such an inhalation means can include, but is not limited to, an inhaling mask. It is preferred that the inhaling mask simultaneously covers the mouth and nose of a subject so that administration to the subject at a suitable concentration is realized.

In one embodiment of the present invention, the pharmaceutical composition of the present invention is provided in a form that may be administered to the subject as-is. As an example, in this embodiment, the pharmaceutical composition of the present invention is provided in a mixed gas form prepared by premixing hydrogen gas and inert gas, as well as oxygen gas for breathing and any other gases at suitable concentrations.

In another aspect of the present invention, the pharmaceutical composition of the present invention is provided in a form that is prepared immediately before administration or at the time of administration to a subject. As an example, in this embodiment, the pharmaceutical composition of the present invention is provided by connecting a container containing a mixed gas of hydrogen gas and inert gas and a container containing oxygen gas to an inhaling mask via a piping, and sending these gases to a patient at a flow rate that allows a concentration suitable for administration to a subject. In one aspect of the present invention, said container may be, in addition to a portable gas cylinder, for example the form of a large-scale storage tank installed outdoors. Moreover, said gas may be contained in the container in a compressed gas form, or may be contained in a liquid gas container (LGC) in liquified from such as liquified hydrogen gas. Moreover, as another example of the present invention, said hydrogen gas may each be supplied from a hydrogen gas generation apparatus. Such a generation apparatus can include those comprising any hydrogen generation means well-known to those skilled in the art, and such a hydrogen generation means can include, but is not limited to, a hydrogen generation means utilizing water electrolysis (such as purified water and alkali water such as potassium hydroxide), a hydrogen generation means utilizing a hydrogen absorbing alloy (such as magnesium and vanadium), a hydrogen generation means utilizing heating or degassing of water in which hydrogen is dissolved, a hydrogen generation means utilizing ammonia degradation, a hydrogen generation means utilizing steam reforming of hydrocarbons (such as methane), a hydrogen generation means utilizing methanol/ethanol reforming, a hydrogen generation means utilizing degradation of water by a catalyst (such as titanium oxide), a hydrogen generation means utilizing the chemical reaction between water and metal hydrides (such as alkaline earth metal hydrides and alkali metal hydrides, typically magnesium hydride), and the like. In the present invention, those comprising a hydrogen generation means utilizing water electrolysis is particularly preferred.

In another aspect of the present invention, the pharmaceutical composition of the present invention is provided by supplying hydrogen gas to a sealed chamber so that gas concentration is maintained constant. As an example, in this embodiment, the pharmaceutical composition of the present invention is provided by supplying a mixed gas consisting of hydrogen gas and inert gas to the sealed chamber in which the subject is present at a flow rate so that the hydrogen concentration in said sealed chamber is maintained at a suitable concentration.

The pharmaceutical composition of the present invention is effective for any human subject after onset of hemorrhagic shock, but the administration subject is not limited to humans, and for example it can also be applied to rodents such as mice, rats, rabbits, and non-human mammals such as monkeys, cows, horses, and goats.

The timing for administering the pharmaceutical composition of the present invention is not particularly limited, and it may be any of a subject in a state of hemorrhagic shock and a subject after withdrawal from hemorrhagic shock. Preferably, the pharmaceutical composition of the present invention is administered to a subject in a state of hemorrhagic shock.

The pharmaceutical composition of the present invention is continuously administered to subject over a certain period of time. The administration period of the pharmaceutical composition of the present invention is not particularly limited as long as it is a period that can exert the effect of improving and/or stabilizing circulatory dynamics after onset of hemorrhagic shock by the pharmaceutical composition of the present invention, and those skilled in the art can appropriately set a suitable period according to the severity of the patient, the injury etc. responsible for hemorrhagic shock, the time from onset of hemorrhage shock to initiation of administration, sex, age, and the like. Such a period may be, but is not limited to, for example at least 10 minutes, at least 30 minutes, at least 1 hour, at least 2 hours, at least 3 hours, at least 4 hours, or longer.

Moreover, the number of doses for the pharmaceutical composition of the present invention is not restricted, and administration can be multiple times. The dose interval and number of doses for the pharmaceutical composition of the present invention can be appropriately set to a suitable dose interval and number of doses according to the symptoms of the patient.

The pharmaceutical composition of the present invention can be provided by a device for providing the pharmaceutical composition of the present invention. The device of the present invention comprises at least a means for providing hydrogen gas, typically a container that contains hydrogen gas or said hydrogen gas generation apparatus, and hydrogen gas which is the active ingredient of the pharmaceutical composition of the present invention is provided by said container or hydrogen gas generation apparatus.

The device of the present invention preferably further comprises a piping connected at one end to said means for providing hydrogen gas. Said piping is a hydrogen gas circulation means for delivering hydrogen gas to the gas inhalation subject, and the other end is either directly connected to an inhalation means for inhaling gas, or connected to a gas mixing device for mixing with other gases such as oxygen gas. In a preferred embodiment, a means for providing hydrogen gas further comprises a control means for monitoring and adjusting the amount of hydrogen gas supplied to the subject. Alternatively, the piping connected to said means for providing hydrogen gas may further comprise said control means.

The device of the present invention preferably further comprises a gas mixing device. The gas mixing device is a means for mixing hydrogen with other gases so that the hydrogen gas from said means for providing hydrogen gas is at a concentration suitable for administration to a subject, and is typically connected to a container containing oxygen gas or an oxygen gas generation apparatus via a piping. In a preferred embodiment, said gas mixing device further comprises a means for monitoring and adjusting the hydrogen concentration in the mixed gas, a means for monitoring and adjusting the oxygen concentration in the mixed gas, and/or a control means for monitoring and adjusting for the flow rate of the mixed gas to the subject.

In another embodiment, the device of the present invention can be used in combination with an artificial ventilator. In this embodiment, said mixed gas device and an artificial ventilator are connected, and the oxygen gas sent from the artificial ventilator is either mixed with hydrogen gas at the gas mixing device to enter the inhalation line, or mixed with hydrogen gas introduced into the inhalation line and then returned to the artificial ventilator as exhaled gas.

The terms used herein are employed for describing particular embodiments, and do not intend to limit the invention.

Moreover, the term "comprising" as used herein, unless the content clearly indicates to be understood otherwise, intends the presence of the described items (such as components, steps, elements, or numbers), and does not exclude the presence of other items (such as components, steps, elements, and numbers).

All of the disclosures of the literatures cited herein should be deemed as cited herein, and those skilled in the art can cite the related disclosed contents in these prior art literatures as a part of the present specification according to the context herein without departing from the spirit and scope of the present invention.

The present invention will now be more specifically described by showing Examples. The present invention is not to be limited in any way by the Examples shown below.

### Examples

### [Example 1]

### (1. Test overview)

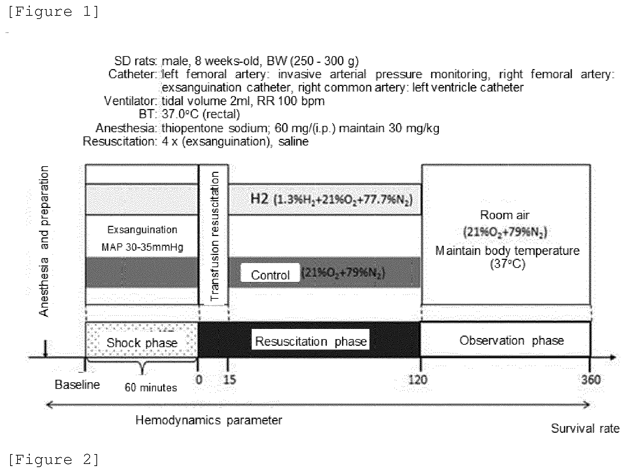

In this Example, the effect of hydrogen gas on a hemorrhagic shock model was evaluated according to the test protocol shown in Figure 1.

With SD rats (male, 250 - 300 g), an anesthetic drug was intraperitoneally administered to perform general anesthesia. Subsequently, a PE catheter was inserted into the left femoral artery to perform direct arterial pressure monitoring, and further, an exsanguination catheter from the right femoral artery and a left ventricle catheter for cardiac function measurement from the right common artery were each inserted.

The shock phase was initiated with the initiation of exsanguination. Exsanguination was slowly performed over 15 minutes, and for a total of 60 minutes since the initiation of exsanguination, the mean arterial pressure was maintained at 30 - 35 mmHg.

The resuscitation phase was initiated with the initiation of resuscitation by transfusion. Transfusion resuscitation by four times the exsanguinated volume of saline was performed over 15 minutes. Test gas inhalation (hydrogen group (21% oxygen + 1.3% hydrogen) and control group (21% oxygen)) were performed for a total of 3 hours from the initiation of exsanguination to 2 hours after the initiation of resuscitation. In doing so, cardiac function (LVEDP, ± dP/dt) during the same period was also recorded.

The initiation of the observation phase was 2 hours after the initiation of the resuscitation phase, and observation was performed for 4 hours thereafter (6 hours from the initiation of the resuscitation phase). During this period, blood pressure (systolic, diastolic, mean), heart rate, and the like were measured with artificial respiration in room air.

Lactic acid value and blood gas at the initiation point of shock phase (baseline), the initiation point of resuscitation phase (0 minute), the initiation point of observation phase (120 minutes), and the end-of-test timepoint (360 minutes) were each measured and evaluated.

### (2. Evaluation of exsanguinated volume)

Table 1 shows the body weight of rats (n = 15 per group), the exsanguinated volume, the exsanguinated volume per 100 g of body weight, and the transfusion resuscitation volume employed in this test. H₂ and Control show hydrogen inhalation group and hydrogen non-inhalation (control) group, respectively. The body weight of rat employed in this test were not different for each group. On the other hand, for the exsanguinated volume, a statistically larger result was shown for the hydrogen inhalation group (control group vs hydrogen inhalation group: 2.29 ± 0.43 vs 2.60 ± 0.40 ml/100 g; p = 0.048). This result means that the hydrogen inhalation group required a larger exsanguinated volume in order to maintain the blood pressure at 30 - 35 mmHg. In other words, it is strongly suggested that circulatory dynamics is stabilized against hypotension by some kind of influence from hydrogen inhalation inside the body of rat.

**[Table 1]**

| | Control (N = 15) | H₂ (N = 15) | p value |
|---|---|---|---|
| Body weight (g) | 274.0 ± 13.0 | 270.0 ± 18.1 | 0.493 |
| Exsanguinated (ml) | 6.27 ± 1.19 | 7.03 ± 1.15 | 0.100 |
| Exsanguinated /body weight (ml/100 g) | 2.29 ± 0.43 | 2.60 ± 0.40 | 0.048 |
| Resuscitation volume (ml) | 25.1 ± 4.8 | 28.8 ± 5.1 | 0.100 |
| resuscitation volume/body weight (ml/100 g) | 9.1 ± 1.7 | 10.4 ± 1.6 | 0.048 |

### (3. Evaluation of survival rate)

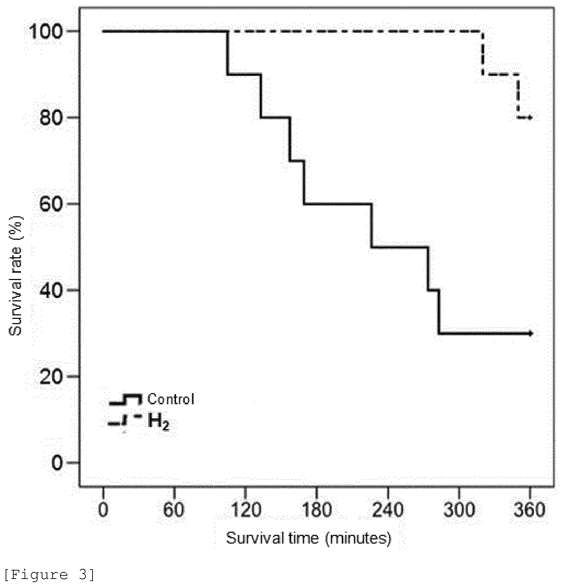

Figure 2 is a graph showing the comparison of survival rate between hydrogen inhalation group and control group. The time from the initiation of resuscitation is shown in the horizontal axis. It is seen that deceased rats start to increase at approximately 2 hours after the initiation of resuscitation in the control group, while the hydrogen inhalation group retains survival.

Ultimately, at the end-of-test timepoint at 6 hours, survival of 8 individuals (8/10) was seen in the hydrogen inhalation group, while only 3 individuals (3/10) could survive in the control group. This result shows that hydrogen inhalation can clearly improve the survival rate after hemorrhagic shock.

### (4. Evaluation of blood pressure)

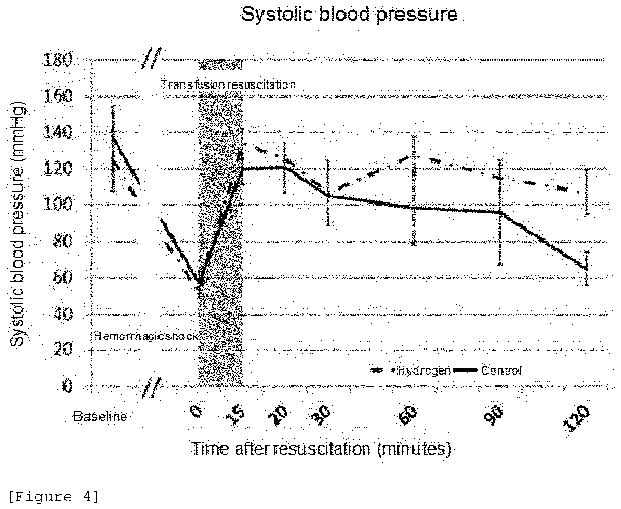

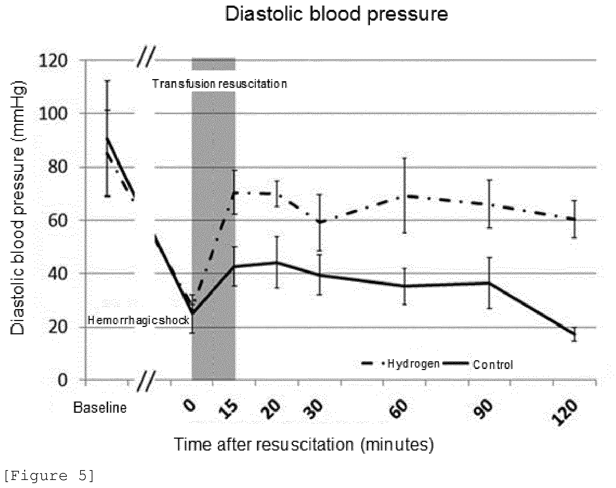

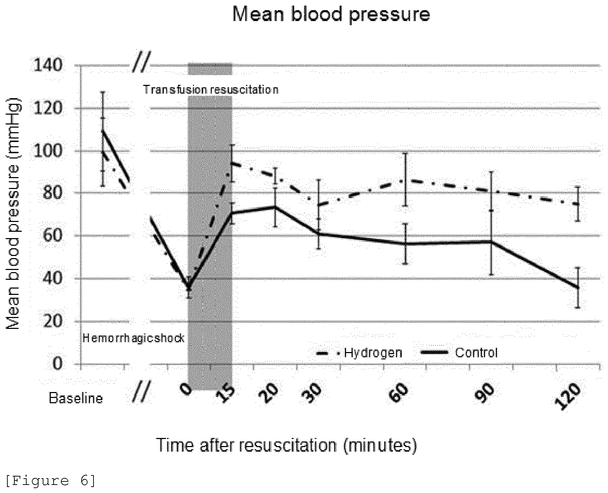

Figures 3 - 5 show the change over time of the systolic blood pressure, the diastolic blood pressure, and the mean blood pressure after the initiation of resuscitation, respectively. As seen from the results, all of the systolic blood pressure, the diastolic blood pressure, and the mean blood pressure show a higher value for the hydrogen inhalation group throughout the observation period. Moreover, the rats in the hydrogen inhalation group maintain increased blood pressure after transfusion resuscitation, whereas the blood pressure declined after a certain amount of time has passed in the control group.

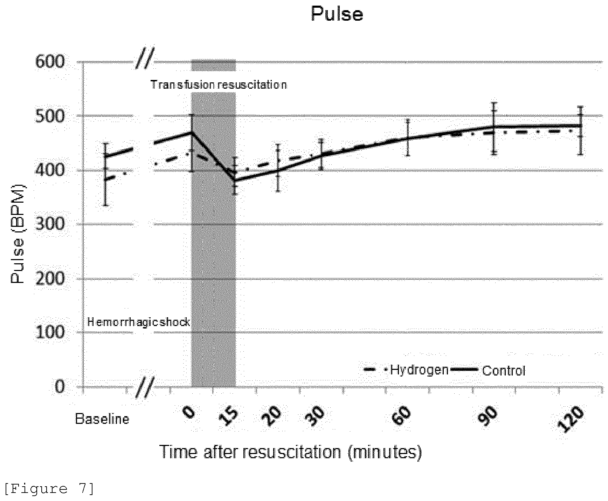

On the other hand, no great difference was seen between the two groups for the heart rate (Figure 6).

### (5. Evaluation of circulatory failure parameters)

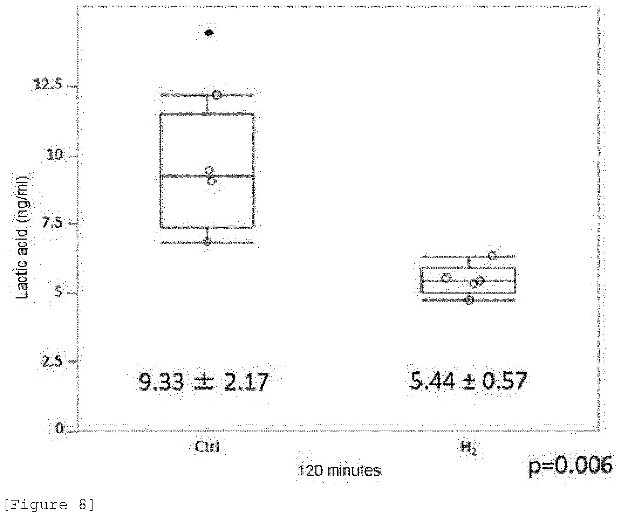

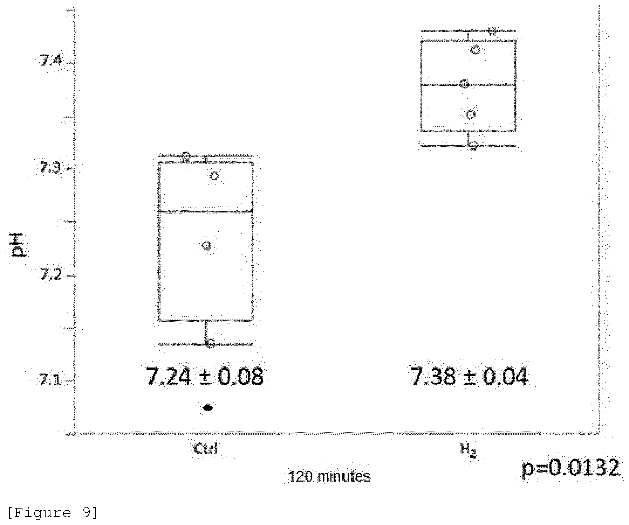

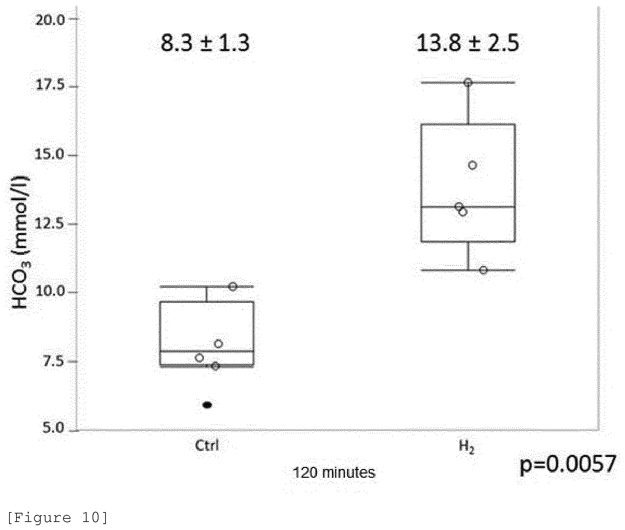

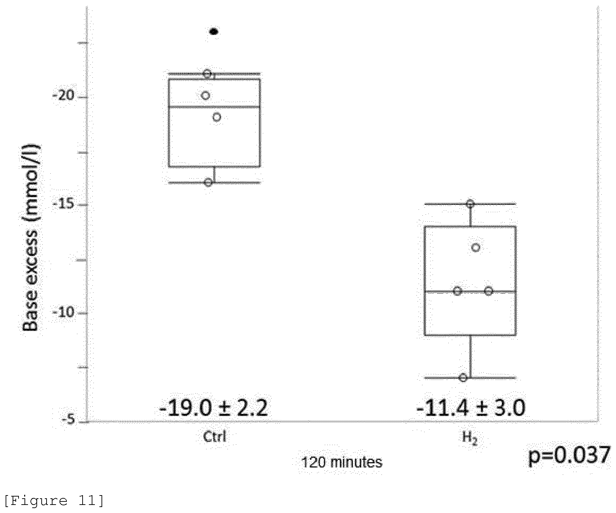

Figures 7 - 10 show the measurement results of circulatory failure parameters at the initiation point of observation phase (120 minutes). The normal value for lactic acid value is 1.0 or less, and it is seen that the value is statistically significantly lower in the hydrogen inhalation group (Figure 7). Moreover, the normal value for pH is about 7.4, and it is seen that the pH is statistically significantly higher in the hydrogen inhalation group and closer to the normal value (Figure 8). Moreover, the normal value for HCO3 is about 24, and it is seen that HCO3 is statistically significantly higher in the hydrogen inhalation group and closer to the normal value (Figure 9). Further, the normal value for BE (base excess) is about -2, and it is seen that the BE value is statistically significantly improved in the hydrogen inhalation group (Figure 20).

All of these results show that circulatory dynamics is statistically significantly stabilized in the hydrogen inhalation group compared to the control group.

### (6. Evaluation of electrolytes and anemic parameter)

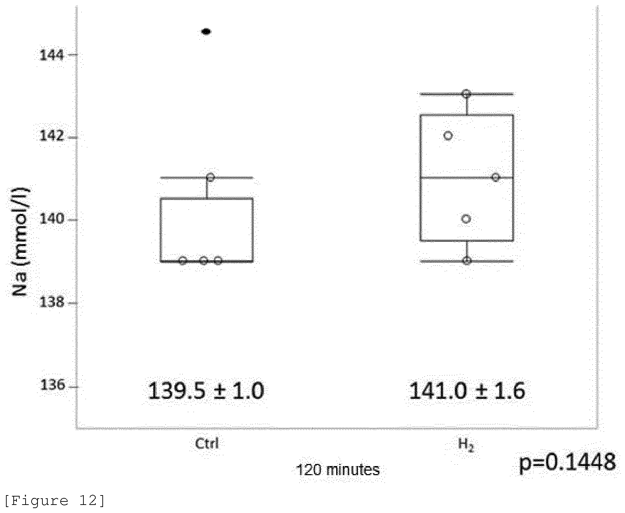

Figures 11 - 13 show the electrolytes (Na and K) and the extent of anemia (hemoglobin (Hb) at the initiation point of observation phase (120 minutes).

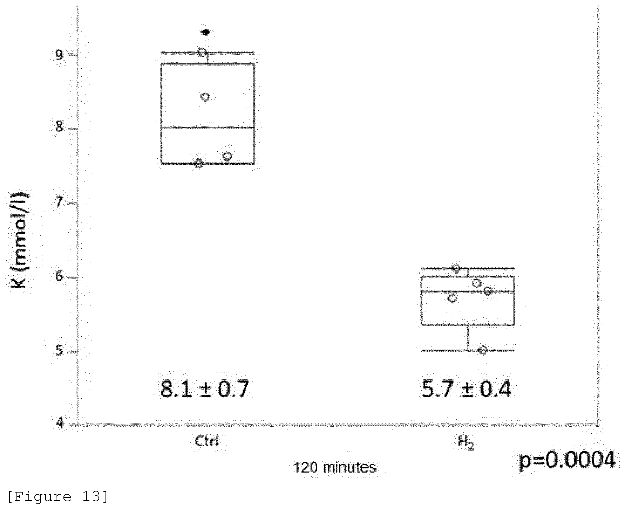

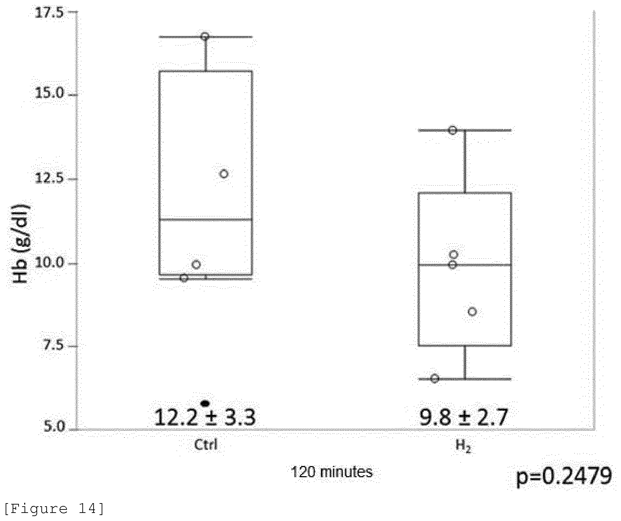

Although no statistically significant difference was seen between the two groups for Na and Hb (Figures 11 and 13), it was shown that K was statistically significantly lower in the hydrogen inhalation group (Figure 12). This result is thought to be the influence of peripheral circulatory failure and renal dysfunction, and it was suggested that there is an organ protective effect by circulatory dynamics stabilization in the hydrogen inhalation group.

### (7. Evaluation of oxygenation indicator)

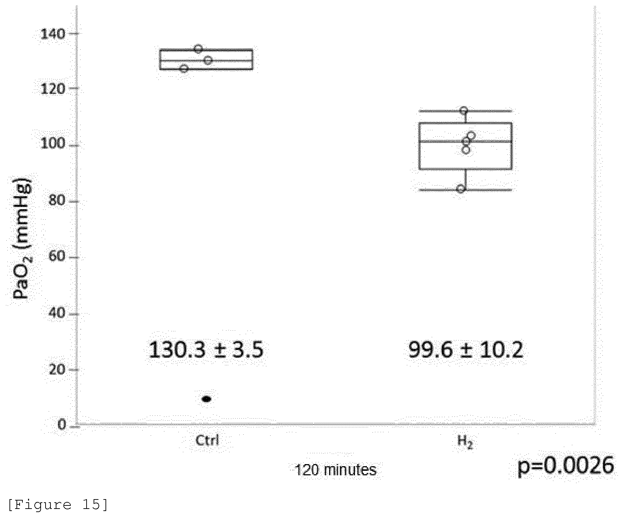

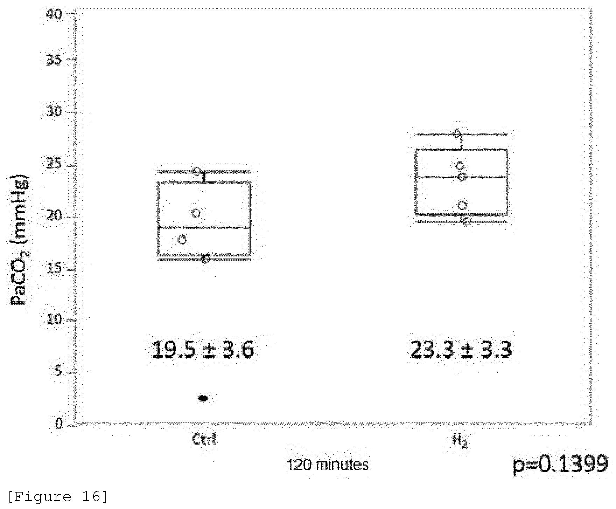

Figures 14 and 15 show oxygenation indicators at the initiation point of observation phase (120 minutes). The normal value for each indicator is around 100 mmHg for PaO2 (Partial pressure of oxygen) and around 40 mmHg for PaCO2 (Partial pressure of carbon dioxide).

PaO2 was shown to have higher values for the control group (Figure 14), but all values were close to the normal value and no clinically significant effect was seen. Moreover, no statistical difference was seen between the two groups for the PaCO2 value (Figure 15). This result shows that respiratory disorder after hemorrhagic shock is not directly involved in the decline in the survival rate in the control group.

### (8. Evaluation of cardiac function)

In the intensive care area, there is a concept of delivery 02 (DO2), the total amount of oxygen delivered to cells throughout the body. Decline of DO2 leads to deterioration in parameters of peripheral metabolic failure such as lactic acid and pH. DO2 is defined by "oxygen saturation (respiratory function)" x "cardiac output volume (cardiac function: circulatory dynamics)" x "hemoglobin." In this test, since no significant difference was seen between the two groups for oxygen saturation and hemoglobin, the influence of hydrogen on improvement of peripheral metabolic failure is thought to be related to cardiac function. Cardiac function consists of three factors: "preload" x "cardiac contractility" x "afterload."

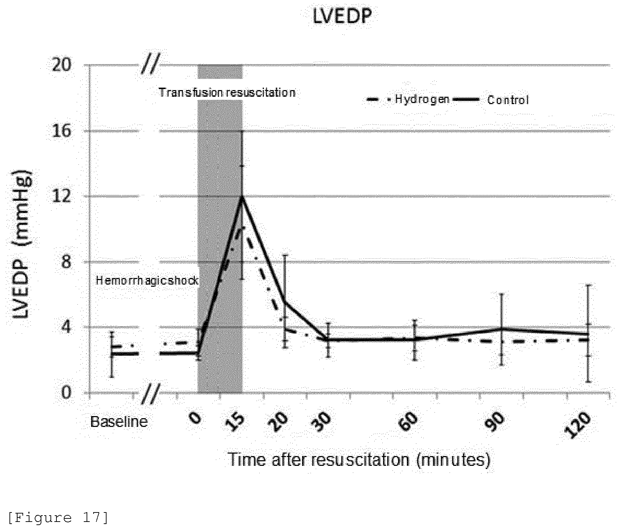

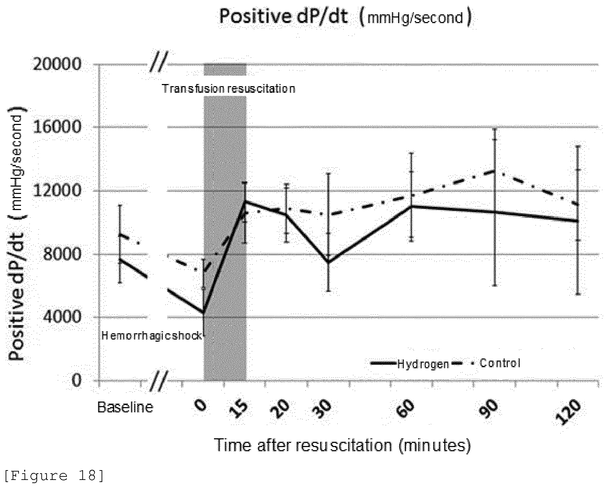

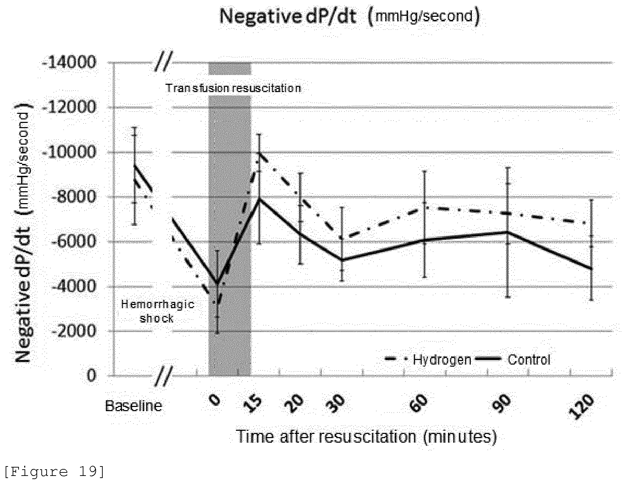

Figures 16 - 18 show the results of left ventricular end-diastolic pressure (LVEDP), positive dP/dt, and negative dP/dt, respectively. LVEDP is a value that is said to be elevated when cardiac contractility declines and cardiac failure is occurring. Positive dP/dt is a sensitive numeric value of the contractile ability of cardiac contractility, and negative dP/dt is a value that reflects the diastolic ability among cardiac contractility.

As seen from the results of Figures 16 - 18, no statistically significantly difference was seen between the two groups for any of LVEDP, positive dP/dt, and negative dP/dt. This result shows that hydrogen gas has almost no influence on the "cardiac contractility," and the difference in cardiac function is due to the influence on the "preload" or "afterload."

"Preload" is defined by exsanguination and transfusion, and as seen from the description in Table 1, since there is only about 1 ml/100 g difference between the two groups and it can be evaluated that there is almost no difference in preload, it is thought that the effect of improving cardiac function by hydrogen mainly acts on the afterload. Afterload represents vascular resistance, and it is thought that hydrogen gas influences the tonus (tension) of the blood vessel to adjust the suitable afterload and stabilize circulatory dynamics.

### [Example 2]

In this test, improvement of the survival rate by hydrogen inhalation at the initiation point of resuscitation after shock in rat hemorrhagic shock model was evaluated.

The procedure in SD rats (250 - 300 g) to exsanguinate so that mean arterial pressure of 30 - 35 mmHg was maintained for 60 minutes, and then resuscitate with four times the exsanguinated volume of saline over 15 minutes to evaluate the survival rate at 6 hours after initiation of resuscitation was according to [Example 1].

In this test, test gas was given by inhalation as follows. Shock phase (-60 minutes to 0 minute): control gas (26% oxygen + 74% nitrogen) Resuscitation phase (0 minute to 120 minutes): hydrogen gas (1.3% hydrogen + 26% oxygen + 72.7% nitrogen) Observation phase (120 minutes to 360 minutes): room air (21% oxygen + 79% nitrogen)

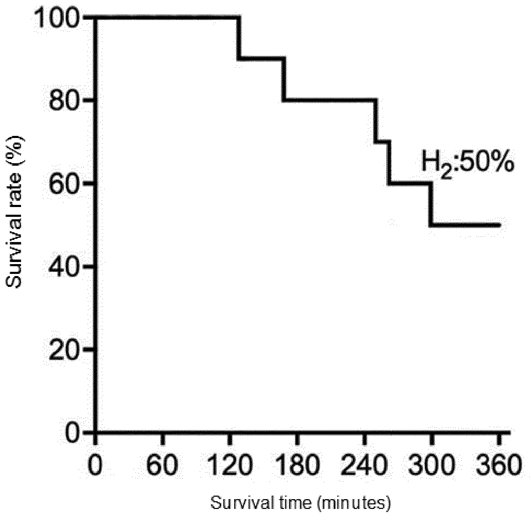

The survival rate at 6 hours after initiation of resuscitation was 50% (Figure 19). This result shows that a survival rate greater than the control group was seen even when hydrogen gas was given by inhalation from the initiation point of resuscitation after onset of hemorrhagic shock (vs 30%; see Figure 2 for the control). This suggests that circulatory dynamics may be improved or stabilized and the survival rate may be improved even when hydrogen gas could not be given by inhalation from the point of hemorrhagic shock, for example even when inhalation of hydrogen gas is initiated from the initiation point of resuscitation after onset of hemorrhagic shock. In real-world clinical practice, this result has clinically an extremely significant meaning in that for example even if one lapses into hemorrhagic shock at the traffic accident site, increase in the survival rate can be anticipated if hydrogen gas could be given by inhalation after being transported to a medical institution.

### Industrial Applicability

According to the pharmaceutical composition of the present invention, circulatory dynamics after onset of hemorrhagic shock can be improved. The pharmaceutical composition of the present invention is extremely useful in that it can increase the survival rate of a subject after hemorrhagic shock or extend the survival time even when a curative therapy for hemorrhagic shock, for example blood transfusion therapy cannot be given.

## Claims

1. A gaseous pharmaceutical composition for use in improving and/or stabilizing circulatory dynamics after onset of hemorrhagic shock, **characterized in that** said pharmaceutical composition comprises hydrogen gas.

2. The pharmaceutical composition for use according to claim 1, **characterized in that** said pharmaceutical composition is administered when blood transfusion therapy cannot be given to a subject.

3. The pharmaceutical composition for use according to claim 1 or 2, **characterized in that** said subject is a subject to which fluid therapy is given.

4. The pharmaceutical composition for use according to claim 3, **characterized in that** said fluid therapy is given prior to, at the same time with, or after administering said composition.

5. The pharmaceutical composition for use according to any one of claims 1 to 4, **characterized in that** the survival rate after onset of hemorrhagic shock is improved by administration of said pharmaceutical composition.

6. The pharmaceutical composition for use according to any one of claims 1 to 4, **characterized in that** the survival time after onset of hemorrhagic shock is extended by administration of said pharmaceutical composition.

7. The pharmaceutical composition for use according to any one of claims 1 to 6, **characterized in that** said improvement and/or stabilization of circulatory dynamics is the maintenance of blood pressure or the maintenance of blood supply to an essential organ based on improvement and/or stabilization of cardiac function or vascular function.

8. The pharmaceutical composition for use according to any one of claims 1 to 7, **characterized in that** said improvement and/or stabilization of cardiac function or vascular function is suppression of cell death in blood vessels, the heart, or other organs under a hypoxic condition.

9. The pharmaceutical composition for use according to any one of claims 1 to 8, **characterized in that** said pharmaceutical composition further comprises oxygen gas.

10. The pharmaceutical composition for use according to any one of claims 1 to 9, **characterized in that** said pharmaceutical composition further comprises an inert gas.

11. The pharmaceutical composition for use according to any one of claims 1 to 10, **characterized in that** the concentration of said hydrogen in said pharmaceutical composition is 0.1% - 4.0% (v/v).

12. The pharmaceutical composition for use according to any one of claims 1 to 11, **characterized in that** the concentration of said hydrogen in said pharmaceutical composition is 1.0% - 2.0% (v/v).

13. The pharmaceutical composition for use according to any one of claims 1 to 12, **characterized in that** said hydrogen gas is provided by a hydrogen gas generation apparatus or a container that contains hydrogen gas.

14. The pharmaceutical composition for use according to claim 13, **characterized in that** said hydrogen gas generation apparatus comprises a hydrogen generation means by water electrolysis.

15. The pharmaceutical composition for use according to claim 13, **characterized in that** said container contains a mixed gas of hydrogen and nitrogen gases.

16. The pharmaceutical composition for use according to any one of claims 13 to 15, **characterized in that** said hydrogen gas generation apparatus or said container further comprises a control means for monitoring and adjusting the amount of hydrogen gas supplied to the subject, on said device or the container itself or a piping connected to said device or container.

17. The pharmaceutical composition for use according to claim 1, wherein said pharmaceutical composition is used for promoting improvement or stabilization of circulatory dynamics after onset of hemorrhagic shock by fluid therapy, and the survival rate after onset of hemorrhagic shock is improved by administration of said pharmaceutical composition and/or the survival time after onset of hemorrhagic shock is extended by administration of said pharmaceutical composition.

18. The pharmaceutical composition for use according to claim 1, wherein said pharmaceutical composition is used for extending the survival time after onset of hemorrhagic shock.

19. The pharmaceutical composition for use according to claim 1, wherein said pharmaceutical composition is used for improving the survival rate after onset of hemorrhagic shock.

## Patentansprüche

1. Gasförmige pharmazeutische Zusammensetzung zur Verwendung beim Verbessern und/oder Stabilisieren der Kreislaufdynamik nach dem Einsetzen eines hämorrhagischen Schocks, **dadurch gekennzeichnet, dass** die genannte pharmazeutische Zusammensetzung Wasserstoffgas umfasst.

2. Pharmazeutische Zusammensetzung zur Verwendung nach Anspruch 1, **dadurch gekennzeichnet, dass** die genannte pharmazeutische Zusammensetzung verabreicht wird, wenn einem Subjekt keine Bluttransfusionstherapie gegeben werden kann.

3. Pharmazeutische Zusammensetzung zur Verwendung nach Anspruch 1 oder 2, **dadurch gekennzeichnet, dass** das genannte Subjekt ein Subjekt ist, dem eine Flüssigkeitstherapie gegeben wird.

4. Pharmazeutische Zusammensetzung zur Verwendung nach Anspruch 3, **dadurch gekennzeichnet, dass** die genannte Flüssigkeitstherapie vor, gleichzeitig mit oder nach der Verabreichung der genannten Zusammensetzung gegeben wird.

5. Pharmazeutische Zusammensetzung zur Verwendung nach einem der Ansprüche 1 bis 4, **dadurch gekennzeichnet, dass** die Überlebensrate nach dem Einsetzen eines hämorrhagischen Schocks durch Verabreichen der genannten pharmazeutischen Zusammensetzung verbessert wird.

6. Pharmazeutische Zusammensetzung zur Verwendung nach einem der Ansprüche 1 bis 4, **dadurch gekennzeichnet, dass** die Überlebenszeit nach dem Einsetzen des hämorrhagischen Schocks durch Verabreichen der genannten pharmazeutischen Zusammensetzung verlängert wird.

7. Pharmazeutische Zusammensetzung zur Verwendung nach einem der Ansprüche 1 bis 6, **dadurch gekennzeichnet, dass** die genannte Verbesserung und/oder Stabilisierung der Kreislaufdynamik die Aufrechterhaltung des Blutdrucks oder die Aufrechterhaltung der Blutversorgung eines essentiellen Organs auf der Basis einer Verbesserung und/oder Stabilisierung der Herzfunktion oder Gefäßfunktion ist.

8. Pharmazeutische Zusammensetzung zur Verwendung nach einem der Ansprüche 1 bis 7, **dadurch gekennzeichnet, dass** die genannte Verbesserung und/oder Stabilisierung der Herzfunktion oder Gefäßfunktion die Unterdrückung von Zelltod in Blutgefäßen, dem Herzen oder anderen Organen unter einer hypoxischen Bedingung ist.

9. Pharmazeutische Zusammensetzung zur Verwendung nach einem der Ansprüche 1 bis 8, **dadurch gekennzeichnet, dass** die genannte pharmazeutische Zusammensetzung ferner Sauerstoffgas umfasst.

10. Pharmazeutische Zusammensetzung zur Verwendung nach einem der Ansprüche 1 bis 9, **dadurch gekennzeichnet, dass** die genannte pharmazeutische Zusammensetzung ferner ein Inertgas umfasst.

11. Pharmazeutische Zusammensetzung zur Verwendung nach einem der Ansprüche 1 bis 10, **dadurch gekennzeichnet, dass** die Konzentration des genannten Wasserstoffs in der genannten pharmazeutischen Zusammensetzung 0,1% - 4,0% (v/v) beträgt.

12. Pharmazeutische Zusammensetzung zur Verwendung nach einem der Ansprüche 1 bis 11, **dadurch gekennzeichnet, dass** die Konzentration des genannten Wasserstoffs in der genannten pharmazeutischen Zusammensetzung 1,0 % - 2,0 % (v/v) beträgt.

13. Pharmazeutische Zusammensetzung zur Verwendung nach einem der Ansprüche 1 bis 12, **dadurch gekennzeichnet, dass** das genannte Wasserstoffgas durch eine Wasserstoffgas-Erzeugungsvorrichtung oder einen Wasserstoffgas enthaltenden Behälter erzeugt wird.

14. Pharmazeutische Zusammensetzung zur Verwendung nach Anspruch 13, **dadurch gekennzeichnet, dass** die genannte Wasserstoffgas-Erzeugungsvorrichtung ein Mittel zum Erzeugen von Wasserstoff durch Wasserelektrolyse umfasst.

15. Pharmazeutische Zusammensetzung zur Verwendung nach Anspruch 13, **dadurch gekennzeichnet, dass** der genannte Behälter ein Mischgas aus Wasserstoff- und Stickstoffgasen enthält.

16. Pharmazeutische Zusammensetzung zur Verwendung nach einem der Ansprüche 13 bis 15, **dadurch gekennzeichnet, dass** die genannte Wasserstoffgas-Erzeugungsvorrichtung oder der genannte Behälter ferner ein Steuermittel zum Überwachen und Einstellen der dem Subjekt zugeführten Wasserstoffgasmenge an dem genannten Gerät oder dem Behälter selbst oder an einer mit dem genannten Gerät oder dem Behälter verbundenen Rohrleitung umfasst.

17. Pharmazeutische Zusammensetzung zur Verwendung nach Anspruch 1, wobei die genannte pharmazeutische Zusammensetzung zum Fördern der Verbesserung oder Stabilisierung der Kreislaufdynamik nach dem Einsetzen eines hämorrhagischen Schocks durch Flüssigkeitstherapie verwendet wird und die Überlebensrate nach dem Einsetzen eines hämorrhagischen Schocks durch Verabreichen der genannten pharmazeutischen Zusammensetzung verbessert wird und/oder die Überlebenszeit nach dem Einsetzen eines hämorrhagischen Schocks durch Verabreichen der genannten pharmazeutischen Zusammensetzung verlängert wird.

18. Pharmazeutische Zusammensetzung zur Verwendung nach Anspruch 1, wobei die genannte pharmazeutische Zusammensetzung zum Verlängern der Überlebenszeit nach dem Einsetzen eines hämorrhagischen Schocks verwendet wird.

19. Pharmazeutische Zusammensetzung zur Verwendung nach Anspruch 1, wobei die genannte pharmazeutische Zusammensetzung zum Verbessern der Überlebensrate nach dem Einsetzen eines hämorrhagischen Schocks verwendet wird.

## Revendications

1. Une composition pharmaceutique gazeuse utilisée pour améliorer et/ou stabiliser la dynamique circulatoire après le début d'un choc hémorragique, **caractérisée en ce que** ladite composition pharmaceutique comprend de l'hydrogène gazeux.

2. La composition pharmaceutique pour utilisation selon la revendication 1, **caractérisée en ce que** ladite composition pharmaceutique est administrée quand une thérapie par transfusion sanguine ne peut pas être administrée à un sujet.

3. La composition pharmaceutique pour utilisation selon la revendication 1 ou 2, **caractérisée en ce que** ledit sujet est un sujet auquel une thérapie liquidienne est administrée.

4. La composition pharmaceutique pour utilisation selon la revendication 3, **caractérisée en ce que** ladite thérapie liquidienne est administrée avant, en même temps ou après l'administration de la dite composition.

5. La composition pharmaceutique pour utilisation selon une quelconque des revendications 1 à 4, **caractérisée en ce que** le taux de survie après le début d'un choc hémorragique est amélioré par l'administration de ladite composition pharmaceutique.

6. La composition pharmaceutique pour utilisation selon une quelconque des revendications 1 à 4, **caractérisée en ce que** le temps de survie après le début d'un choc hémorragique est prolongé par l'administration of ladite composition pharmaceutique.

7. La composition pharmaceutique pour utilisation selon une quelconque des revendications 1 à 6, **caractérisée en ce que** ladite amélioration et/ou stabilisation de la dynamique circulatoire est le maintien de la pression artérielle ou le maintien de l'apport sanguin à un organe essentiel sur la base de l'amélioration et/ou la stabilisation de la fonction cardiaque ou de la fonction vasculaire.

8. La composition pharmaceutique pour utilisation selon une quelconque des revendications 1 à 7, **caractérisée en ce que** ladite amélioration et/ou stabilisation de la fonction cardiaque ou de la fonction vasculaire est la suppression de la mort cellulaire dans les vaisseaux sanguins, le cœur ou d'autres organes dans un état hypoxique.

9. La composition pharmaceutique pour utilisation selon une quelconque des revendications 1 à 8, **caractérisée en ce que** ladite composition pharmaceutique comprend en sus de l'oxygène gazeux.

10. La composition pharmaceutique pour utilisation selon une quelconque des revendications 1 à 9, **caractérisée en ce que** ladite composition pharmaceutique comprend en sus un gaz inerte.

11. La composition pharmaceutique pour utilisation selon une quelconque des revendications 1 à 10, **caractérisée en ce que** la concentration dudit hydrogène dans ladite composition pharmaceutique est de 0,1% à 4,0% (v/v).

12. La composition pharmaceutique pour utilisation selon une quelconque des revendications 1 à 11, **caractérisée en ce que** la concentration dudit hydrogène dans ladite composition pharmaceutique est de 1,0% à 2,0% (v/v).

13. La composition pharmaceutique pour utilisation selon une quelconque des revendications 1 à 12, **caractérisée en ce que** ledit hydrogène gazeux est fourni par un appareil de génération d'hydrogène gazeux ou un récipient contenant de l'hydrogène gazeux.

14. La composition pharmaceutique pour utilisation selon la revendication 13, **caractérisée en ce que** ledit appareil de génération d'hydrogène gazeux comprend un moyen de génération d'hydrogène par électrolyse de l'eau.

15. La composition pharmaceutique pour utilisation selon la revendication 13, **caractérisée en ce que** ledit récipient contient un mélange gazeux d'hydrogène et d'azote gazeux.

16. La composition pharmaceutique pour utilisation selon une quelconque des revendications 13 à 15, **caractérisée en ce que** ledit appareil de génération d'hydrogène gazeux ou ledit récipient comprennent en sus un moyen de commande pour surveiller et régler la quantité d'hydrogène gazeux fournie au sujet, sur ledit dispositif ou le récipient lui-même ou une tuyauterie reliée audit dispositif ou récipient.

17. La composition pharmaceutique pour utilisation selon la revendication 1, ladite composition pharmaceutique étant utilisée pour favoriser l'amélioration ou la stabilisation de la dynamique circulatoire après le début d'un choc hémorragique par une thérapie liquidienne, et le taux de survie après le début d'un choc hémorragique étant amélioré par l'administration de ladite composition pharmaceutique et/ou le temps de survie après le début d'un choc hémorragique étant prolongé par l'administration de ladite composition pharmaceutique.

18. La composition pharmaceutique pour utilisation selon la revendication 1, ladite composition pharmaceutique étant utilisée pour prolonger le temps de survie après le début d'un choc hémorragique.

19. La composition pharmaceutique pour utilisation selon la revendication 1, ladite composition pharmaceutique étant utilisée pour améliorer le taux de survie après le début d'un choc hémorragique.
